# EUROPÄISCHE PATENTSCHRIFT

(11) **EP 0 431 151 B1**
(45) Veröffentlichungstag und Bekanntmachung des Hinweises auf die Patenterteilung: **15.03.1995**
(21) Anmeldenummer: 90915942.8
(22) Anmeldetag: 07.05.1990
(51) Int. Cl.: C07J 7/00, C07J 43/00

(54) **VERFAHREN ZUR HERSTELLUNG VON 21-BROM-4-PREGNEN-3,20-DION-DERIVATEN**
METHOD FOR PREPARING 21-BROM-4-PREGNEN-3,20-DIONE DERIVATIVES
PROCEDE POUR PREPARER DES DERIVES DE 21-BROME-4-PREGNENE-3,20-DIONE

(30) Priorität: 12.05.1989 DE 3915951
(43) Veröffentlichungstag der Anmeldung: 12.06.1991
(73) Patentinhaber: SCHERING AKTIENGESELLSCHAFT, 59192 Bergkamen (DE)
(72) Erfinder: HEMPEL, Gerhard, verstorben (DE)
(86) Internationale Anmeldenummer: DE9000329
(87) Internationale Veröffentlichungsnummer: WO9013558

(56) Entgegenhaltungen:
- CHEMICAL ABSTRACTS, Band 95, Nr. 5, 3. August 1981, (Columbus, Ohio, US), sieheseite 797* Zusammenfassung Nr. 43465j, & JP-A-8122797 (Joint Center forChemistry, Sofia) 3. Marz 1981*

## Beschreibung

Die Erfindung betrifft ein Verfahren zur Herstellung von 21-Brom-4-pregnen-3,20-dion-Derivaten der allgemeinen Formel I 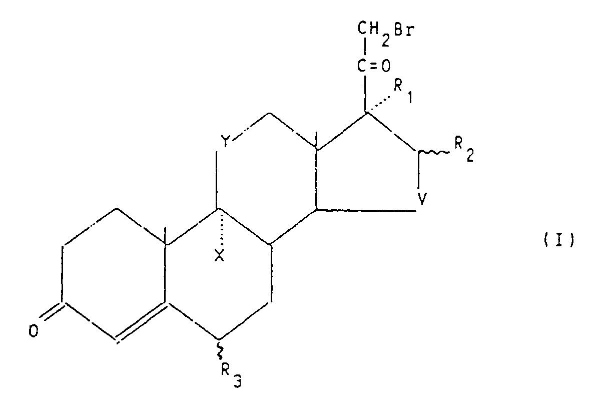
worin
- R₁: ein Wasserstoffatom oder eine Hydroxygruppe bedeutet,
- R: ₂ ein Wasserstoffatom oder eine Methylgruppe symbolisiert oder worin
- R₁ und R₂: gemeinsam eine Isopropylidendioxygruppe darstellen,
worin
- R₃: ein Wasserstoffatom, ein Fluoratom oder eine Methylgruppe darstellt,
- V: eine Methylengruppe oder Ethylengruppe bedeutet,
- X: ein Wasserstoffatom, ein Fluoratom oder ein Chloratom darstellt und
- Y: eine Methylengruppe, Hydroxymethylengruppe oder eine Carbonylgruppe bedeutet, durch Bromierung eines Enamins der allgemeinen Formel II 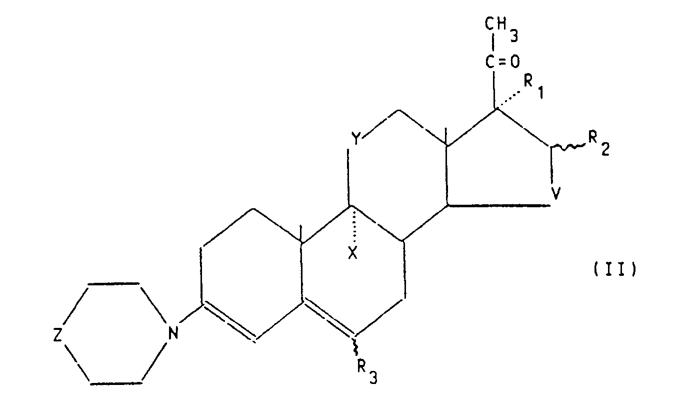
worin
R₁, R₂, R₃, X, Y und V die obengenannte Bedeutung besitzen und Z eine Kohlenstoff-Kohlenstoffbindung, eine Methylengruppe oder ein Sauerstoffatom darstellt mit elementarem Brom in einem niederen Alkohol mit maximal 4 Kohlenstoffatomen, welches dadurch gekennzeichnet ist, daß man der Reaktionsmischung vor der Bromierung pro mol Steroid 0,01 bis 1 mol Methansulfonsäure und 1 bis 2 mol eines Ortho-Esters der allgemeinen Formel III

R₄C(OR₅)₃ (III)

worin,
- R₄: ein Wasserstoffatom oder einen 1 bis 4 Kohlenstoffatome enthaltenden Alkylrest und
- R₅: eine 1 bis 4 Kohlenstoffatome enthaltende Alkylgruppe bedeuten, umsetzt.

Die 21-Brom-4-pregnen-3,20-dion-Derivate der allgemeinen Formel I sind bekanntlich wertvolle Zwischenprodukte, die beispielsweise zur Herstellung von 21-Acyloxy-4-pregnen-3,20-dion-Derivaten der allgemeinen Formel IV 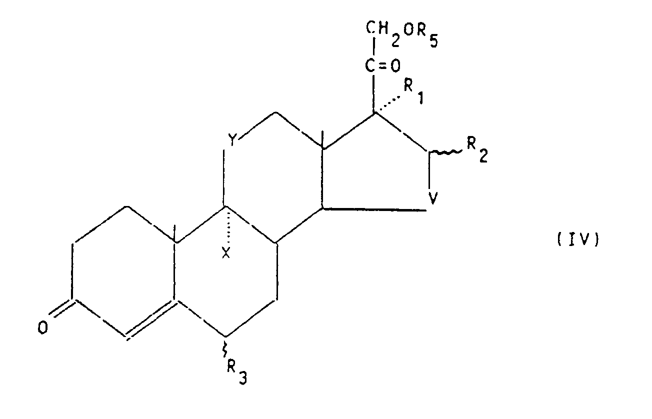
worin
- R₁, R₂, R₃, V, X und Y: die obengenannte Bedeutung besitzen und
- R₅: eine Alkanoylgruppe mit bis zu 6 Kohlenstoffatomen oder eine Benzoylgruppe darstellt, verwendet werden können.

Es ist bekannt, daß man diese 21-Brom-4-pregnen-3,20-dion-Derivate durch Bromieren von Enaminen der allgemeinen Formel II hergestellt werden können, welche ihrerseits aus 4-Pregnen-3,20-dion-Derivaten der allgemeinen Formel V 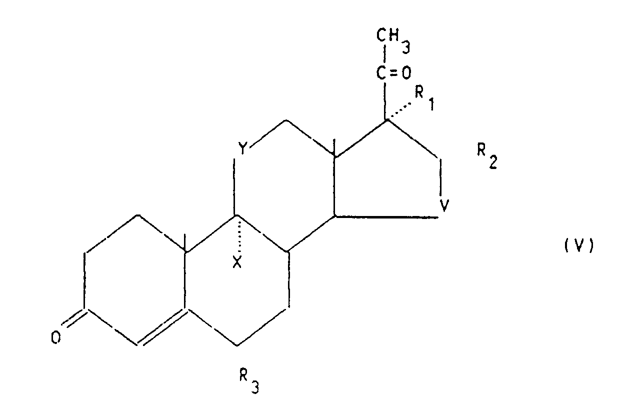
worin
R₁, R₂, R₃, V, X und Y die obengenannte Bedeutung besitzen synthetisiert werden.

Da sowohl die Enamine der allgemeinen Formel II als auch die hieraus hergestellten 21-Brom-4-pregnen-3,20-dion-Derivate der allgemeinen Formel I wenig stabil und demzufolge nicht lagerfähig sind, führt man die Synthese in der Praxis fast stets so durch, daß man diese Verbindungen unmittelbar nach ihrer Darstellung weiter umsetzt.

So ist im Beispiel 1 der DE-B 18 01 410 beschrieben, daß man 17α-Hydroxy-4-pregnen-3,20-dion in einem 4-stufigen Verfahren in 21-Acetoxy-17α-hydroxy-4-pregnen-3,20-dion überführen kann. Laut Patentschrift erzielt man bei diesem Verfahren eine Ausbeute von 66 % der Theorie. Die Praxis zeigt aber, daß die erreichbare Ausbeute doch wesentlich geringer ist und 55 % der Theorie kaum übersteigt. Zudem beträgt die Reinheit des erhaltenen Verfahrensprodukts knapp 90 % und ist somit unbefriedigend.

Ein etwas verbessertes Verfahren wurde von B. Gadsby et. al (John Fried and John A. Edwards "Organic Reactions in Steroid Chemistry, Vol 2, van Nostrand Reinhold Comp. New York etc., 1972, p 223) entwickelt. Nach diesem Verfahren wird, wie die Praxis zeigt, 17α-Hydroxy-4-pregnen-3.20-dion in einer Ausbeute von 62 % in 21-Acetoxy-4-pregnen-3,20-dion überführt. Die Reinheit des erhaltenen Produkts betragt 94 %. Ein wesentlicher Mangel dieses Verfahrens ist es, daß die Bromierung selbst bei -60° C durchgeführt wird, wodurch die Reaktion sehr aufwendig wird.

Erwähnenswert ist ferner ein in der japanischen Patentanmeldung 81.22797 (ref. Chem. Abstr. 95, 1981, 43465j) beschriebenes Verfahren, nach dem 17α-Hydroxy-4-pregnen-3,20-dion mit einer Ausbeute von 67 % der Theorie in das 21-Acetoxy-17α-hydroxy-4-pregnen-3,20-dion (Reinheit 95 %) überführt werden kann, wie die Praxis zeigt. Nachteilig bei diesem Verfahren ist, daß die Bromierung in Gegenwart von Perchlorsäure durchgeführt wird, wodurch die Reaktionsmischung sehr explosionsgefährdet wird. Weitere Nachteile dieses Verfahrens sind, daß die Bromierung in sehr hoher Verdünnung durchgeführt wird und recht viel Zeit beansprucht (7-8 Stunden).

Mithilfe des erfindungsgemäßen Verfahrens gelingt es, wie in den Ausführungsbeispielen näher erläutert wird, die Ausgangsprodukte der allgemeinen Formel V mit einer Ausbeute über 90 % der Theorie in Produkte der allgemeinen Formel IV (Reinheit über 93 %) zu überführen. Weitere Vorteile des erfindungsgemäßen Verfahrens sind darin zu sehen, daß die Bromierung in relativ konzentriert er Lösung etwa bei Raumtemperatur in relativ kurzer Zeit durchgeführt werden kann, ohne daß die Verwendung explosionsgefährdeter Stoffe erforderlich wäre.

Es wurde bereits erwähnt, daß die als Ausgangssubstanzen für das erfindungsgemäße Verfahren dienenden Enamine der allgemeinen Formel II ihrerseits aus den 4-Pregnen-3,20-dion-Derivaten der allgemeinen Formel V hergestellt werden. Besonders geeignete 4-Pregnen-3,20-dion-Derivate der allgemeinen Formel V, welche als Vorprodukte zur Durchführung des erfindungsgemäßen Verfahrens dienen können sind solche, die als Substituenten R₁ eine Hydroxygruppe und/oder als Substituenten R₂ ein Wasserstoffatom oder eine Methylgruppe und/oder als Substituenten R₃ ein Wasserstoffatom oder eine Methylgruppe und/oder als Substituenten X und Y jeweils Wasserstoffatome und/oder als Gruppierung V eine Methylengruppe besitzen.

Die Herstellung der Enamie der allgemeinen Formel V erfolgt in bekannter Weise; zum Beispiel in dem man die 4-Pregnen-3,20-dion-Derivate in einem niederen Alkohol (wie Methanol, Ethanol, Propanol oder Isopropanol mit Piperidin, Morpholin oder vorzugsweise Pyrrolidin auf 40-70° C erwärmt. Nach erfolgter Reaktion kristallisieren die Enamine beim Abkühlen der Reaktionsmischung aus und können ohne weitere Reinigung weiter umgesetzt werden.

Zur Umsetzung der Enamine der allgemeinen Formel II werden diese zweckmäßigerweise in einem niederen Alkohol - wie Methanol, Ethanol, Propanol oder Isopropanol - suspendiert, die Suspension mit etwa 1,0 bis 2.0 mol Methansulfonsäure pro mol Steroid versetzt und auf ca. 40 bis 70° C erwärmt bis eine klare Lösung entsteht. Dann setzt man der Lösung 0,01 bis 1 mol eines Orthoesters der allgemeinen Formel III - vorzugsweise Orthoameisensäuretrimethylester oder Orthomeisensäuretriethylester - pro mol Steroid zu und tropft innerhalb von 30 bis 180 Minuten bei etwa 0 bis 50° C die berechnete Menge Brom - gewünschtenfalls in einen niederen Alkohol gelöst - zu.

Nach erfolgter Bromierung gibt man zur Reaktionsmischung einen Uberschuß an Kaliumkarbonat - vorzugsweise in Wasser gelöst - zu rührt die Mischung noch 30 bis 180 Minuten und fällt das 21-Brom-4-pregnen-3,20-dion-Derivat der allgemeinen Formel I durch Eingießen in Eiswasser. Die so erhaltenen 21-Brom-4-pregnen-3,20-dion-Derivate der allgemeinen Formel I können anschließend in bekannter Weise in die 21-Acyloxy-4-pregnen-3,20-dion-Derivate der allgemeinen Formel IV überführt werden, indem man sie in Aceton oder Methanol mit einem Kaliumacylat - vorzugsweise Kaliumacetat - 1 bis 5 Stunden lang unter Rückfluß erhitzt und dann das gebildete Verfahrenspro` dukt durch Eingießen in Eiswasser abscheidet.

Die nachfolgenden Ausführungsbeispiele dienen zur näheren Erläuterung des erfindungsgemäßen Verfahrens.

### Beispiel 1

a) Eine Suspension von 2,365 kg 17α-Hydroxy-4-pregnen-3,20-dion in 20 l Methanol wird unter Rühren und Stickstoff-Begasung auf 55° C erwärmt. Dann setzt man der Mischung 0,946 l Pyrrolidin zu, rührt noch eine Stunde lang bei 55° C, kühlt sie auf -5° C, saugt das abgeschiedene 17α-Hydroxy-3-(N-pyrrolidinyl)-3,5-pregnadien-20-on ab und wäscht es mit eiskaltem Methanol.
b) Das methanolfeuchte 17α-Hydroxy-3-(N-pyrrolidinyl)-3,5-pregnadien-20-on wird bei Raumtemperatur unter Rühren und Stickstoff-Begasung in 12 l Methanol suspendiert, mit 0,563 l Methansulfonsäure versetzt und erwärmt bis eine klare Lösung entsteht. Dann läßt man die Reaktionsmischung auf Raumtemperatur erkalten, versetzt sie mit 0,017 l Orthoameisensäuretrimethylester und versetzt sie innerhalb von 90 Minuten mit einer Lösung von 1,202 kg Brom in 5,5 l Methanol. Man rührt noch weitere 30 Minuten und versetzt sie mit einer Lösung von 2,157 kg Kaliumkarbonat in 5,7 l Wasser. Man rührt weitere 2 Stunden lang bei Raumtemperatur. Dann neutralisiert man die Reaktionsmischung mit Essigsäure, fällt das 21-Brom-17α-hydroxy-4-pregnen-3,20-dion durch Eingießen in Eiswasser aus und wäscht es mit Wasser.
c) Das noch feuchte 21-Brom-17α-hydroxy-4-pregnen-3,20-dion wird unter Rühren und Stickstoff-Begasung in 24 l Aceton eingetragen, mit 1,64 kg Kaliumacetat und 0,024 l Essigsäure versetzt und 210 Minuten unter Rückfluß erhitzt. Dann destilliert man 7,5 l Aceton ab, läßt die Reaktionsmischung auf Raumtemperatur erkalten, fällt das Reaktionsprodukt durch Eingießen in Eiswasser aus, wäscht es mit Wasser und trocknet es im Vakuum bei 50° C. Man erhält so 2,617 kg 21-Acetoxy-17α-hydroxy-4-pregnen-3,20-dion vom Schmelzpunkt 232-335° C.Ausbeute = 94 % der Theorie. Reinheit des Produkts 96,4 %.

### Beispiel 2

a) Eine Suspension von 40,00 g 17a-Hydroxy-16β-methyl-4-pregnen-3,11,20-trion in 90 ml Methanol wird unter Rühren und Stickstoff-Begasung auf 55° C erwärmt. Dann setzt man der Mischung 16 ml Pyrrolidin zu, rührt noch eine Stunde lang bei 55° C, kühlt sie auf 0° C, saugt das abgeschiedene 17α-Hydroxy-16β-methyl-3(N-pyrrolidinyl)-3,5-pregnadien-11,20-dion ab und wäscht es mit eiskaltem Methanol.
b) Das methanolfeuchte 17α-Hydroxy-16β-methyl-3-(N-pyrrolidinyl)-3,5-pregnadien-11,20-dion wird bei Raumtemperatur unter Rühren und Stickstoff-Begasung in 590 ml Methanol suspendiert, mit 8,17 ml Methansulfonsäure versetzt und erwärmt bis eine klare Lösung entsteht. Dann läßt man die Reaktionsmischung auf Raumtemperatur erkalten, versetzt sie mit 0,67 ml Orthoameisensäuretrimethylester und versetzt sie innerhalb von 120 Minuten mit einer Lösung von 16,45 ml Brom in 72,6 ml Methanol. Man rührt noch weitere 15 Minuten und versetzt sie mit einer Lösung von 16.34 g Kaliumkarbonat in 72,6 ml Wasser. Man rührt eine weitere Stunde lang bei Raumtemperatur. Dann neutralisiert man die Reaktionsmischung mit Essigsäure, und erhält eine Suspension des 21-Brom-17α-hydroxy-16β-methyl-4-pregnen-3,11,20-trions.
c) Die Suspension des 21-Brom-17α-hydroxy-16β-methyl-4-pregnen-3,11,20-trions wird unter Rühren mit 61,74 g Kaliumacetat und 90 Minuten auf 50° C erwärmt. Dann destilliert man das Methanol weigehend ab, läßt die Reaktionsmischung auf Raumtemperatur erkalten, saugt das Reaktionsprodukt ab, wäscht es mit Wasser und trocknet es im Vakuum bei 50° C. Man erhält so 42,9 g 21-Acetoxy-17α-hydroxy-16β-methyl-4-pregnen-3,11,20-trion vom Schmelzpunkt 214-219° C. Ausbeute = 92 % der Theorie. Reinheit des Produkts 93,8 %.

## Patentansprüche

1. Verfahren zur Herstellung von 21-Brom-4-pregnen-3,20-dion-Derivaten der allgemeinen Formel I 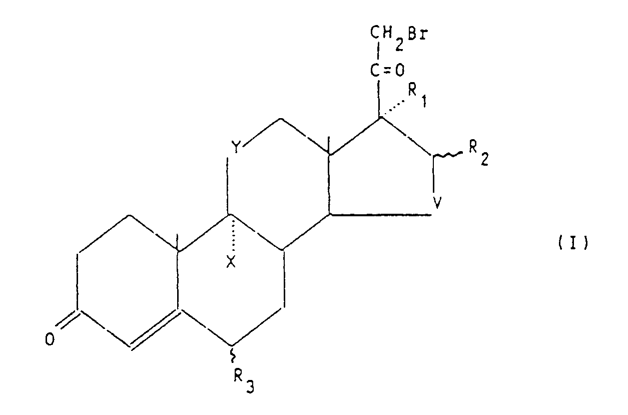 worin
R₁ ein Wasserstoffatom oder eine Hydroxygruppe bedeutet,
R₂ ein Wasserstoffatom oder eine Methylgruppe symbolisiert oder worin
R₁ und R₂ gemeinsam eine Isopropylidendioxygruppe darstellen,
worin
R₃ ein Wasserstoffatom, ein Fluoratom oder eine Methylgruppe darstellt,
V eine Methylengruppe oder Ethylengruppe bedeutet,
X ein Wasserstoffatom, ein Fluoratom oder ein Chloratom darstellt und
Y eine Methylengruppe, Hydroxymethylengruppe oder eine Carbonylgruppe bedeutet, durch Bromierung eines Enamins der allgemeinen Formel II 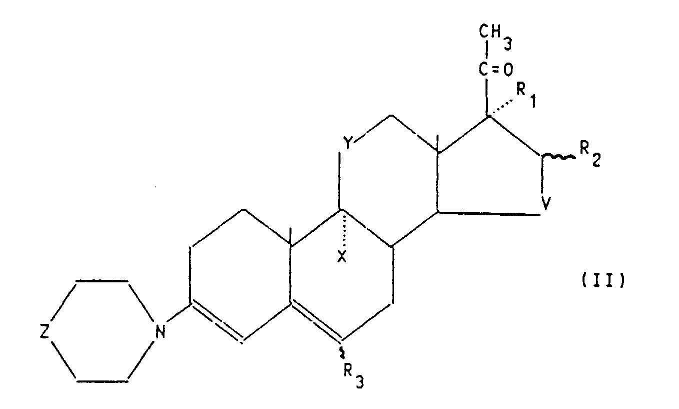
worin
R₁, R₂, R₃, X, Y und V die obengenannte Bedeutung besitzen und
Z eine Kohlenstoff-Kohlenstoffbindung, eine Methylengruppe oder ein Sauerstoffatom darstellt mit elementarem Brom in einem niederen Alkohol mit maximal 4 Kohlenstoffatomen, dadurch gekennzeichnet, daß man der Reaktionsmischung vor der Bromierung pro mol Steroid 1 bis 2 mol Methansulfonsäure und 0,01 bis 1 mol eines Ortho-Esters der allgemeinen Formel III
R₄C(OR₅)₃ (III)
worin,
R₄ ein Wasserstoffatom oder einen 1 bis 4 Kohlenstoffatome enthaltenden Alkylrest und
R₅ eine 1 bis 4 Kohlenstoffatome enthaltende Alkylgruppe bedeuten, umsetzt.

## Claims

1. Process for the preparation of 21-bromo-4-pregnen-3,20-dione derivatives of the general formula I 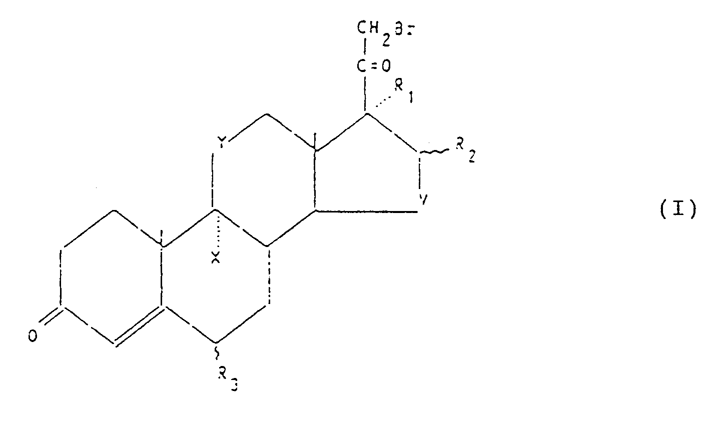 wherein
R₁ represents a hydrogen atom or a hydroxy group,
R₂ represents a hydrogen atom or a methyl group, or
R₁ and R₂ together represent an isopropylidenedioxy group,
R₃ represents a hydrogen atom, a fluorine atom or a methyl group,
V represents a methylene group or an ethylene group,
X represents a hydrogen atom, a fluorine atom or a chlorine atom, and
Y represents a methylene group, a hydroxymethylene group or a carbonyl group,
by bromination of an enamine of the general formula II 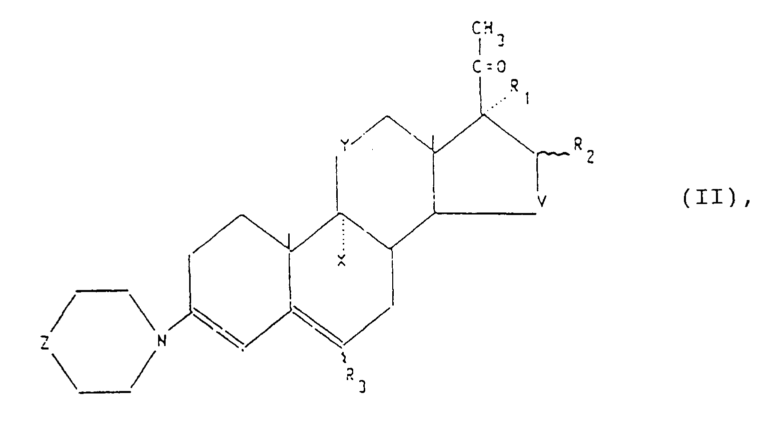 wherein R₁, R₂, R₃, X, Y and V have the meaning given above and Z represents a carbon-carbon bond, a methylene group or an oxygen atom, with elemental bromine in a lower alcohol having not more than four carbon atoms, characterised in that, before the bromination, there are added to the reaction mixture per mol of steroid from 1 to 2 mol of methanesulphonic acid and from 0.01 to 1 mol of an ortho-ester of the general formula III
R₄C(OR₅)₃ (III),
wherein
R₄ represents a hydrogen atom, or an alkyl radical containing from 1 to 4 carbon atoms, and
R₅ represents an alkyl group containing from 1 to 4 carbon atoms.

## Revendications

1. Procédé pour préparer des dérivés de la 21-bromo-4-prégnène-3,20-dione de formule générale I 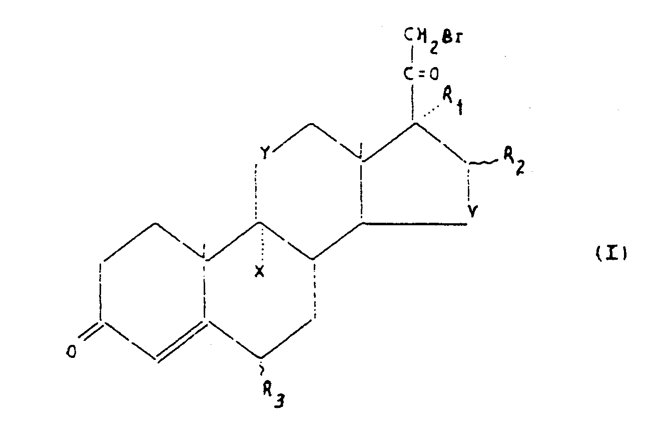 dans laquelle
R₁ représente un atome d'hydrogène ou un groupe hydroxy,
R₂ représente un atome d'hydrogène ou un groupe méthyle, ou dans laquelle
R₁ et R₂ représentent ensemble un groupe isopropylidènedioxy,
dans laquelle
R₃ représente un atome d'hydrogène, un atome de fluor ou un groupe méthyle,
V représente un groupe méthylène ou un groupe éthylène,
X représente un atome d'hydrogène, un atome de fluor ou un atome de chlore, et
Y représente un groupe méthylène, a groupe hydroxyméthylène ou un groupe carbonyle,
par bromation d'une énamine de formule générale II 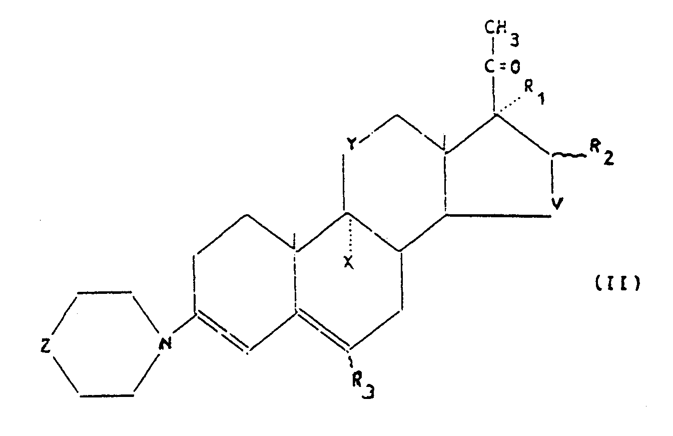 dans laquelle R₁, R₂, R₃, X, Y et V ont la signification précédente, et
Z représente une liaison carbone-carbone, un groupe méthylène ou un atome d'oxygène, avec du brome élémentaire dans un alcool inférieur avec au maximum 4 atomes de carbone,
le procédé étant caractérisé en ce que, avant la bromation, on fait réagir le mélange réactionnel avec, par mole de stéroïde, de 1 à 2 moles d'acide méthane-sulfonique et de 0,01 à 1 mole d'un ortho-ester de formule générale III
R₄C(OR₅)₃ (III)
dans laquelle
R₄ représente un atome d'hydrogène ou un reste alkyle comportant de 1 à 4 atomes de carbone, et
R₅ représente un groupe alkyle ayant de 1 à 4 atomes de carbone.
